# EUROPEAN PATENT SPECIFICATION

(11) **EP 0 580 185 B1**
(45) Date of publication and mention of the grant of the patent: **31.03.1999**
(21) Application number: 93113704.6
(22) Date of filing: 15.08.1989
(51) Int. Cl.: C07C 69/157, C07C 69/96, A61K 31/265, A61K 31/22, C07C 67/08, C07C 68/06, C07C 50/32, A61K 31/12

(54) **New Naphthoquinones and their use as medicaments**
Neue Naphthochinone und ihre Anwendung als Arzneimittel
Nouvelles naphtoquinones et leur application comme médicaments

(30) Priority: 16.08.1988 GB 8819477; 16.08.1988 GB 8819478; 16.08.1988 GB 8819479; 16.08.1988 GB 8819480
(43) Date of publication of application: 26.01.1994
(62) Divisional of application: 89308268.5
(73) Proprietor: THE WELLCOME FOUNDATION LIMITED, London NW1 2BP (GB)
(72) Inventor: Latter, Victoria, The Wellcome Res. Lab., Beckenham, Kent BR3 3BS (GB); Gutteridge, Winston Edward, The Wellcome Res. Lab., Beckenham, Kent BR3 3BS (GB); Hudson, Alan Thomas, The Wellcome Res. Lab., Beckenham, Kent BR3 3BS (GB)
(74) Representative: Stott, Michael John

(56) References cited:
- No relevant documents disclosed

## Description

The present invention relates to certain novel naphthoquinones of formula (VI). More specifically, the invention is concerned with their use in the treatment and/or prophylaxis of malaria.

Pneumocystis carinii is a parasite which has a natural habitat in lung tissue. In a host with a normal immune system P.carinii is not considered to be pathogenic. However, when the immune system is defective P.carinii is liable to cause pneumonia. There is a variety of circumstances in which the immune system may be defective or deficient. Thus, for example immune system deficiency is common in immature or premature infants (neonates). It may also result from suppression by certain drugs, which may be deliberate e.g. in certain patients receiving organ transplants, or unavoidable e.g. as a side-effect of cancer chemotherapy. Disordered growth of one or more constituent parts of the immune system. e.g. as in certain forms of cancer, may also result in immunodeficiency.

Immune deficiency may furthermore be caused by viral infections, including human immunodeficiency virus (HIV). It has been reported (Hughes, W.T. (1987) Treatment and Prophylaxis of Pneumocystis carinii pneumonia. Parasitology Today 3(11) 332-335) that at least 60% of patients with acquired immunodeficiency syndrome (AIDS) suffer from Pneumocystis carinii pneumonia.

In this specification the term "immunocompromised host" will be used to describc hosts with a deficient or defective immune system.

Without treatment. Pneumocystis carinii pneumonia is almost always fatal in immunocompromised hosts. The most widely used treatments for this condition are trimethoprim-sulphamethoxazole (cotrimoxaole) and pentamidine. However, both of these treatments have been reported to be only around 50-70% effective in AIDS patients and to produce a much higher than usual incidence of adverse reactions (about 50%) (Wofsy, C.B. Antimicrobial Agents Annual. 1986. Vol 1, p377-400). There is thus a need for new agents. especially for the prophylaxis of P.carinii pneumonia.

A wide range of naphthoquinones is known in the art. Such compounds have been variously described as having antimalarial, anticoccidial and antitheilerial activity. Some compounds have also been described as possessing activity against external parasites. Thus, Fieser et al, J. Amer. Chem. Soc. 1948, 70, 3156-3165 (and references cited therein) describes a large number of 2-substituted-3-hydroxy-1,4-naphthoquinones as having antimalarial activity. A number of these compounds have also been described in U.S. Patent Specification No. 2 553 648. Further classes of 2-substituted-3-hydroxy-1,4-naphthoquinones having activity as antimalarial, anticoccidial and/or antitheilerial agents are described in U.S. Patents Nos. 3 367 830, and 3 347 742, U.K. Patent Specification No. 1553424, and European Patent Specifications Nos. 2 228, 77551 and 77550. European Patent Application No. 123239 discloses synergistic combinations of anti-protozoal naphthoquinones and 4-pyridinols or alkanoic esters thereof, which are said to be especially useful for the treatment or prophylaxis of malaria.

European Patent No. 123,238 discloses 2-substituted-3-hydroxy-1,4-naphthoquinones of formula (I) 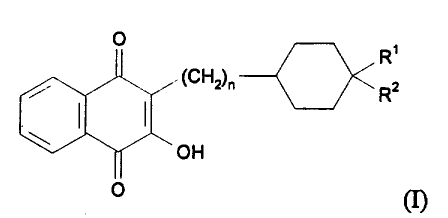 wherein either R¹ is hydrogen and R² is selected from C₁₋₆ alkoxy, aralkoxy, C₁₋₆ alkyl-C₁₋₆ alkoxy, phenyl substituted by one or two groups selected from halogen and C1-6 alkyl, halogen and perhalo-C₁₋₆ alkyl or R¹ and R² are both C₁₋₆ alkyl or phenyl, and n is zero or 1, and physiologically acceptable salts thereof. Compounds of formula (I) wherein n is zero are said to be active against the human malaria parasite Plasmodium falciparum and also against Eimeria species such as E.tenella and E.acervulina, which are causitive organisms of coccidiosis. Compounds of formula (I) where n is 1 are said to be active against protozoa of the genus Theileria, in particular T.annulata and T.parva.

We have now found that a variety of naphthoquinones are active in vivo against Pneumocystis carinii pneumonia infections in rats. Activity has also been demonstrated in an in vitro preparation of P. carinii.

Prevention of P.carinii infections is particularly important in an immunocompromised host, as discussed hereinabove. In the case of immunosuppression resulting from HIV infection, prophylaxis may be required by those diagnosed as seropositive for HIV. and those with progressive generalised lymphadenopathy (PGL) or AIDS-related complex (ARC) as well as patients suffering from AIDS.

In a first aspect. the present invention provides novel compounds of formula (VI) 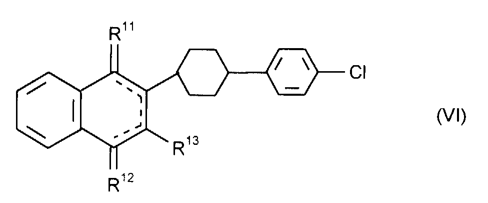 wherein
R¹¹ and R¹² each represent =O and the dotted line represents a double bond between the 2 and 3 positions of the quinone ring, in which case R¹³ represents:
a group OCOR⁵, wherein R⁵ is a C₁₋₁₀alkyl group, a C₃₋₁₀ cycloalkyl group, a C₁₋₁₀alkoxy group, or a phenyl or naphthyl group, each such R⁵ group being optionally substituted e.g. by amino, mono-or di-C₁₋₄alkylamino, carboxy or hydroxy; or
a group OR⁶ or SR⁶, wherein R⁶ is an optionally substituted C₁₋₁₀alkyl, C₃₋₁₀ cycloalkyl, phenyl or naphthyl group as defined for R⁵; or
a group NR⁷R⁸, wherein R⁷ and R⁸ each independently represent hydrogen or C₁₋₄alkyl, or the group NR⁷R⁸ represents a 5-7 membered saturated heterocyclic ring, which may optionally contain a further heteroatom selected from nitrogen, oxygen or sulphur;
or the dotted line represents double bonds at the 1,2 and 3,4 positions of the quinol ring and R¹¹, R¹² and R¹³ each represents a group -OCOR¹⁴, wherein R¹⁴ represents an optionally substituted C₁₋₁₀alkyl group.

Compounds of formula (VI) have been found to exhibit activity in vitro against the parasite Plasmodium falciparum and in vivo against the parasite Plasmodium yoelii as illustrated hereinafter. These compounds may therefore be useful in the treatment and/or prophylaxis of malaria.

It will be appreciated that the compounds formula (VI) may exist as the cis or trans isomer, that is to say that the cyclohexyl ring may be cis or trans substituted by the naphthoquinone nucleus and the substituent on the cyclohexyl ring (in the case of formula (VI) the chlorophenyl group). Both cis and trans isomers and mixtures thereof in any ratio may be used in accordance with the present invention. In general when the compound is in the form of a mixture of isomers the trans isomer will be present in an amount of about 50% or will be the predominant isomer but the use of mixtures in which the cis isomer predominates is also included within the scope of the invention. The specific ratio of isomers may be varied as required; typical mixtures include those in which the cis/trans isomer ratio is about 1:1,40:60 and 5:95. For use in treatment and prophylaxis of Pneumocystis carinii infections the trans isomer of the compound of formula (VI), or a mixture of its cis and trans isomers containing at least 95% e.g. 99% of the trans isomer, is preferred.

A preferred compound of formula (VI) is 2-acetoxy-3-[trans-4-(4-chlorophenyl) cyclohexyl]-1,4-naphthoquinone.

A further preferred compound of formula (VI) is 2-[trans-4-(4-chlorophenyl) cyclohexyl]-1,3,4-triacetoxynaphthalene.

The synthesis of compounds of formula (VI) may be effected by methods already known and described in the chemical literature (for example the patent specifications listed hereinbefore) or by analogous methods. In particular novel compounds of formula (VI) may be prepared by the following methods which form a further aspect of this invention:
(a) reaction of a compound of formula (V) or (VII), with a compound serving to introduce the required group R¹³, and where appropriate the groups R¹¹ and R¹²; 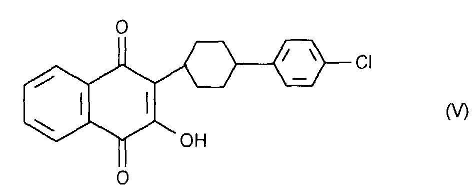 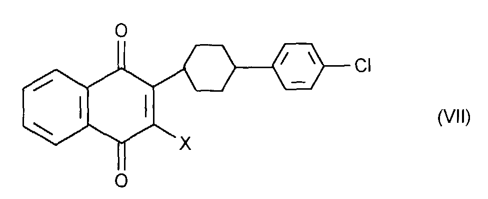
(b) reaction of a compound of formula (VIII): 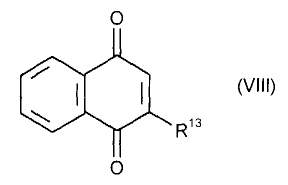 wherein R¹³ is as defined above with a donor compound serving to introduce the 4-(4-chlorophenyl)cyclohexyl group.

With regard to process (a) compounds (VI) wherein R¹¹ and R¹² represent = 0 and R¹³ represents a group OCOR⁵ may be prepared by esterification of the compound (V). Esterification may be effected in conventional manner using the appropriate acid R⁵COOH or acid derivative e.g. an acid anhydride, acid chloride or an activated ester such as an alkylhaloformate e.g. an alkylchloroformate. To prepare a compound of formula (VI) wherein R¹¹, R¹² and R¹³ each represent a group -OCOR¹⁴, the esterification is carried out in the presence of a reducing agent. e.g. zinc.

Compounds of formula (VI) wherein R¹³ is a group OR⁶ or SR⁶ may be prepared from a compound (VII) wherein X is a halogen atom. Thus for example the group OR⁶ may be introduced by reaction with the appropriate alcohol, e.g. methanol or ethanol in the presence of sodium, and the group SR⁶ may be introduced by reaction with the corresponding thiol, R⁶SH.

Compounds of formula (VI) wherein R¹³ is -NR⁷R⁸ may be prepared by reduction of the corresponding compound wherein R¹³ is azido. e.g. using lithium aluminium hydride in tetrahydrofuran, followed where necessary and/or desired by alkylation of the resulting amino group. The azido compound may be prepared from a compound of formula (VII) wherein X is halogen, by reaction e.g. with sodium azide.

Compounds of formula (VII) may be prepared for example in an analogous manner to process (b) described below.

With regard to process (b), a suitable donor compound is the corresponding cycloalkane carboxylic acid which may undergo oxidative decarboxylation. For instance persulphate with a catalyst, such as silver ions. is convenient for the purpose. (c.f.Jacobson, N.. et al., Annalen, 1972, 763, 135 and Acta Chem. Scand, 1973, 27, 3211). Conveniently ammonium persulphate can be used as the oxidising agent, and the catalyst is silver nitrate. Further details of this process are described in EPA 123238. The compound of formula (VIII) used as starting material may be prepared from the corresponding 3-halo compound using methods analogous to process (a).

Hereinafter naphthoquinones active against P.carinii, described by formula (VI), and their physiologically acceptable salts and other physiologically functional derivatives will be referred to as the "naphthoquinone". It will be appreciated that the amount of the naphthoquinone required for use in the treatment or prophylaxis of P.carinii will depend inter alia on the activity of the particular compound, the route of administration, the age and weight of the patient and the severity of the condition being treated. In general, a suitable dose for administration to man for the treatment of P.carinii pneumonia may be in the range of 0.lmg to 200mg per kilogram bodyweight per day, for example from 1mg/kg to 100mg/kg, particularly 10 to 50 mg/kg. For administration by inhalation the dose may conveniently be in the range of 0.1 to 20 mg/kg/day, e.g. 0.5 to 10 mg/kg/day. It will be appreciated that for administration to neonates, lower doses may be required.

For prophylactic treatment the naphthoquinone may also be given less frequently, e.g. as a single dose on alternate days, once or twice per week or once or twice per month. The dosage for prophylactic treatment will depend inter alia on the activity of the naphthoquinone, the frequency of administration, and, where a depot preparation or controlled release formulation is used the rate of release of the active ingredient. Thus for once-weekly administration a suitable prophylactic dose could be in the range 0.05 to 100 mg/kg,e.g. 0.05 to 50 mg/kg particularly 5 to 50 mg/kg.

Suitable dosages of a compound of formula (VI) for the treatment or prophylaxis of malaria in man are also within the ranges given above for the treatment and prophylaxis of P.carinii pneumonia.

For use according to the present invention the naphthoquinone is preferably presented as a pharmaceutical formulation.

Pharmaceutical formulations comprise the naphthoquinone or a physiologically acceptable salt or other physiologically functional derivative thereof together with one or more pharmaceutically acceptable carriers therefor and optionally other therapeutic and/or prophylactic ingredients. The carrier(s) must be acceptable in the sense of being compatible with the other ingredients of the formula and not deleterious to the recipient thereof.

The naphthoquinone may conveniently be presented as a pharmaceutical formulation in unit dosage form. A convenient unit dose formulation contains the naphthoquinone in an amount of from 10 mg to 3g e.g. 10 mg to 1g.

Pharmaceutical formulations include those suitable for oral, topical (including dermal, buccal and sublingual), rectal or parenteral (including subcutaneous, intradermal, intramuscular and intravenous), nasal and pulmonary administration e.g. by inhalation. The formulation may, where appropriate, be conveniently presented in discrete dosage units and may be prepared by any of the methods well known in the art of pharmacy. All methods include the step of bringing into association the naphthoquinone with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of the naphthoquinone. A tablet may be made by compression or moulding, optionally with one or more accessory ingredients. Compressed tablets may be prepared by compressing in a suitable machine the naphthoquinone in a free-flowing form such as a powder or granules optionally mixed with a binder, lubricant, inert diluent, lubricating agent. surface-active agent or dispersing agent. Moulded tablets may be made by moulding an inert liquid diluent. Tablets may be optionally coated and. if uncoated. may optionally be scored. Capsules may be prepared by filling the naphthoquinone, either alone or in admixture with one or more accessory ingredients, into the capsule shells and then sealing them in the usual manner. Cachets are analogous to capsules wherein the naphthoquinone together with any accessory ingredient(s) is sealed in a rice paper envelope. The naphthoquinone compound may also be formulated as dispersible granules, which may for example be suspended in water before administration, or sprinkled on food. The granules may be packaged e.g. in a sachet. Formulations suitable for oral administration wherein the carrier is a liquid may be presented as a solution or a suspension in an aqueous liquid or a non-aqueous liquid, or as an oil-in-water liquid emulsion.

Formulations for oral administration include controlled release dosage forms e.g. tablets wherein the naphthoquinone is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the naphthoquinone with the softened or melted carrier(s) followed by chilling and shaping in moulds.

Pharmaceutical formulations suitable for parenteral administration include sterile solutions or suspensions of the naphthoquinone in aqueous or oleaginous vehicles. Injectible preparations may be adapted for bolus injection or continuous infusion. Such preparations are conveniently presented in unit dose or multi-dose containers which are sealed after introduction of the formulation until required for use. Alternatively, the naphthoquinone may be in powder form which is constituted with a suitable vehicle, such as sterile, pyrogen-free water. before use.

The naphthoquinone may also be formulated as a long-acting depot preparation, which may be administered by intramuscular injection or by implantation e.g. subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing the naphthoquinone and desirably having a diameter in the range 0.5 to 7 microns are delivered into the bronchial tree of the recipient. Such formulations may be in the form of finely comminuted powders which may conveniently be presented in a pierceable capsule, for example of gelatin, for use in an inhalation device, or as a self-propelling formulation (also referred to as an aerosol formulation) comprising the naphthoquinone, a suitable liquid or gaseous propellant and optionally other ingredients such as a surfactant and/or a solid diluent. Suitable liquid propellants include propane and the chlorofluorocarbons. and suitable gaseous propellants include carbon dioxide. Suitable surfactants include sorbitan trioleate (which is available for example under the trade name "Arlacel 85"). Polysorbate 20 and oleic acid. Self-propelling formulations may also be employed wherein the active ingredient is dispensed in the form of droplets of solution or supension. The self-propelling formulation typically contains from 0.05 to 20mg/ml e.g. 0.1 to 5mg/ml of the active ingredient.

Such self-propelling formulations are analogous to those known in the art and may be prepared by established procedures. Suitably they are presented in a container provided with either a manually-operable or automatically functioning valve having the desired spray characteristics: advantageously the valve is of a metered type delivering a fixed volume, for example, 25 to 100 microlitres, upon each operation thereof.

As a further possibility the naphthoquinone may be in the form of a solution or suspension for use in an atomiser or nebuliser whereby an accelerated airstream or ultrasonic agitation is employed to produce a fine droplet mist for inhalation. Such solutions or suspensions may comprise, in addition to the naphthoquinone and solvent(s). optional ingredients such as surfactants. Suitable surfactants include those described above for self-propelling formulations. The solution or suspension typically contains from 0.05 to 20mg/ml e.g. 0.1 to 5mg/ml of the naphthoquinone. When a suspension of the naphthoquinone is employed, this compound is preferably in finely divided form, e.g. in micronised form.

Formulations suitable for nasal administration include presentations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns. for administration by rapid inhalation through the nasal passage from a container held close up to the nose, and nasal drops comprising 0.2 to 5% w/v of the naphthoquinone in aqueous or oily solution or suspension.

It should be understood that in addition to the aforementioned carrier ingredients the pharmaceutical formulations for the various routes of administration described above may include, as appropriate one or more additional carrier ingredients such as diluents. buffers, flavouring agents, binders, surface active agents, thickeners. lubricants, preservatives (including anti-oxidants) and the like, and substances included for the purpose of rendering the formulation isotonic with the blood of the intended recipient.

Novel compounds of formula (VI) may also be formulated in the manner described above for use in the treatment and/or prophylaxis of malaria and such formulations form a further aspect of the present invention.

The above naphthoquinones may also be used in accordance with the present invention in combination or concurrently with other therapeutic agents, for example agents used in the treatment of immunocompromised patients, including anticancer agents such as interferons e.g. alpha-interferons; antiviral agents such as azidothymidine (AZT,zidovudine), immunostimulants and immunodulators. The naphthoquinone may also be administered in combination with a 4-pyridinol compound, as described in EPA 123,239 e.g. 3,5-dichloro-2,6-dimethylpyridinol (meticlorpindol).

The following non-limiting examples illustrate inter alia the following aspects of the present invention:
novel pharmaceutical formulations:
novel compounds of formula (VI).

### Example 1 - Preparation of compound (V)

### 2-[trans-4-(4-Chlorophenyl)cyclohexyl]-3-hydroxy-1,4-naphthoquinone

a) 4-(4-Chlorophenyl)cyclohexane-1-carboxylic Acid
   Acetyl chloride (30g) and finely powdered aluminium chloride (60g) were stirred together in carbon disulphide (120 ml) and then cooled to -50°C, in a CO₂/oxitol bath. Cyclohexene (30 g), previously cooled to -50°C, was added dropwise during 10 minutes while maintaining the temperature of the reaction mixture at below -20°C. The mixture was stirred at -50°C for a further 60 minutes and the solvent then decanted to leave a gummy orange complex. A little chlorobenzene was added as the material warmed to ambient temperature; the remainder of the chlorobenzene (total 300 ml) was then added, the so-obtained solution heated at 40°C for 3 hours with stirring, poured onto a mixture of ice and concentrated hydrochloric acid and the organic layer separated, washed with 2M hydrochloric acid, 2M sodium hydroxide and water. dried over anhydrous sodium sulphate and evaporated to dryness. The product was distilled in vacuo, the fraction boiling at 140-154°C (0.1 mm Hg) collected, diluted with an equal volume of petroleum ether (40-60). cooled to -6°C and a continuous stream of nitrogen gas bubbled through. and the separated colourless solid recovered.
   Bromine (2.8ml) was added to a solution of sodium hydroxide (6.2g) in water (42 ml) at 0°C. The above-obtained substituted hexahydroacetophenone (3.1g) was dissolved in dioxan (15 ml) and the cold hypobromite solution then added, keeping the reaction mixture at below 20°C. The reaction mixture was stirred at ambient temperature for 6 hours then allowed to stand overnight. Sodium metabisulphite was added to destroy excess hypobromite, the mixture cooled and then acidified to give a colourless solid. The solid was filtered off, washed with water, dried and recrystallised from ethanol to give 4-(4-chlorophenyl)cyclohexane-1-carboxylic acid, m.p. 254-256°C.
b) 2-[4-(4-chlorophenyl)cyclohexyl]-3-chloro-1,4-naphthoquinone
   A mixture of 2-chloro-1,4-naphthoquinone (3.95g. 0.02 mol), 4-(4-chlorophenyl)cyclohexane-1-carboxylic acid (4.9g. 0.02 mol) and powdered silver nitrate (1.05g, 0.0062 mol) was heated to reflux with vigorous stirring in 40 ml of acetonitrile. A solution of ammonium persulphate (12.0g, 0.0525 mol) in 50 ml of water was added dropwise over 1 hour. The mixture was refluxed for 3 hours then cooled in ice for 30 mins, after which it was filtered, and the residual sticky solid extracted twice with boiling chloroform to remove inorganic material. The chloroform was removed by evaporation to leave a yellow-brown solid (ca 2.7g). This was dissolved in 40 ml of boiling acetonitrile; a little insoluble material was removed by filtration. On cooling. the title compound separated as yellow crystals. (550 mg) m.p. 172-175°C.
   NMR, δH(d₆-DMSO) 8.05 (2H, mult., β-naphth), 7.85(2H, mult.. α-naphth), 7.30 (4H, s., PhH), 3.30 (1H, br.t., CH), 2.67 (1H, br.t., CH), 1.2-2.4 (8H, mult., 4xCH₂).
c) 2-[4-(4-chlorophenyl)cyclohexyl]-3-hydroxy-1,4-naphthoquinone
   The product of stage (b) was suspended in 10 ml of boiling methanol and 0.55g of potassium hydroxide in 5.5 ml of water was added dropwise over 15 mins. The mixture was refluxed until a dark red solution formed, (after ca. 6 hrs) when 2 ml of concentrated hydrochloric acid was cautiously added dropwise. The mixture was cooled and filtered, and the solid residue washed thoroughly with water. The water washings were re-acidified and filtered. The combined solid residues (500 mg) mp 200-209°, were recrystallised from acetonitrile to give the title product as the trans-isomer (300 mg) m.p. 216-219°C.

### Example 2

### 2-Acetoxy-3-[trans-4-(4-chlorophenyl)cyclohexyl]-1,4-naphthoquinone

The compound of Example 1(c) (1.5g. 0.004mol) was suspended in acetic anhydride (3ml) and a few drops of concentrated sulphuric acid were added with vigorous stirring. A further quantity of acetic anhydride (3ml) was then added, the mixture stirred for 30 minutes and then poured into 15ml of water causing a vigorous reaction. The resulting mixture was cooled in ice and filtered to give pale yellow crystals which were washed with water and dried to give the impure product (1.6g) mp149-152°. This was recrystallised from 100ml of SVM to give the title compound (1.3g) mp158-160°.

NMR, δH (d₆-DMSO) 8.1 (2H, m, β-naphth), 7.9 (2H, m, α-naphth), 7.35 (4H, s, PhH), 3.1 (1H, br.t., CH), 2.6 (1H, br.t., CH), 2.5 (3H, s. COMe), 1.5-2.0 (8H, m, CH₂).

### Example 3

### 2-Ethoxycarbonyloxy-3-[trans-4-(4-chlorophenyl)cyclohexyl]-1,4 naphthoquinone

The compound of Example 1(c) (1.1g, 0.003mol) and pyridine (0.24g, 0.003mol) was stirred in 5ml of toluene and cooled in a water bath while 4ml of ethyl chloroformate was added dropwise over a period of 15 minutes. The mixture was stirred for a further 30 minutes then poured into a mixture of ethyl acetate and water. The organic layer was separated, dried and evaporated to a yellow solid which was recrystallised from chloroform/petrol to give the impure product (850mg) mp145-149°. This material was dissolved in chloroform, washed several times with ice cold 0.1N sodium hydroxide and then water. The organic layer was dried and evaporated to give product (450mg) mp147-9°. The reaction was repeated on the same scale, the product combined with the aforementioned material and then recrystallised from chloroform/petrol to give the title compound (1.3g) mp153-155°.

NMR, δH (d₆-DMSO) 8.1 (2H, m, β-naphth), 7.9 (2H, m, α-naphth), 7.4 (4H, s, PhH), 4.3 (2H, q, OCH₂), 3.1 (1H, br.t., CH), 2.6 (1H, br.t.. CH), 1.5-2.0 (8H, m, CH₂), 1.4 (3H, t, Me).

### Example 4

### 2-[trans-4-(4-Chlorophenyl)cyclohexyl]-3-(4-dimethylaminobenzoyloxy)-1,4-naphthoquinone

The compound of Example 1(c) (2.0g, 5.4mmol) in dry toluene (50ml) containing dry pyridine (0.44g, 1eq) was stirred at around room temperature. 4-Dimethylaminobenzoyl chloride (1g, 1eq) in dry toluene (25ml) was added dropwise over 15 minutes. The mixture was stirred at around room temperature for 1 hour, heated at reflux for 10 hours, left standing for 38 hours, and then refluxed for a further 7 hours. The mixture was then cooled, washed with water, sodium bicarbonate solution and again with water, dried (MgSO₄) and evaporated in vacuo to a yellow solid which was recrystallised from ethanol to give the title compound (1.25g) mp117-121° (shrinks above 113°).

NMR, δH (CDCl₃) 8.1, 6.75 (4H, mult., β-naphth + α-Me₂N-Ph), 7.72 (2H, mult., α-naphth), 7.18 (4H, mult., PhH), 6.72 (2H, d, b-Me₂N-Ph), 3.18 (1H, br.t., CH), 3.14 (6H, s., 2xMe), 2.52 (1H, br.t., CH), 1.4-2.2 (8H, mult., 4xCH₂).

### Example 5

### 2-[trans-4-(4-Chlorophenyl)cyclohexyl]-1,3,4-triacetoxynaphthalene

The compound of Example 1(c) (1.0g) and zinc dust (1.0g) was stirred at room temperature for 24 hours in acetic anhydride (6ml) with one drop TEA. The reaction mixture was filtered and added to water (50ml) and stirred for one hour. The resulting white precipitate was filtered, washed with water (4 x 20ml) and dried to give the title compound (0.4g) mp177-179°.

NMR, δH (d₆-DMSO) 7.87 (2H, mult., β-naphth), 7.63 (2H, mult., α-naphth), 7.35 (4H, s., Ph.H), 2.95 (1H, br.t., CH), 2.64 (1H, br.t., CH), 2.62 (3H, s., OAc), 2.49 (3H, s., OAc), 1.4-2.3 (8H, mult., 4xCH₂).

### Example 6

The following examples illustrate novel pharmaceutical formulations according to the present invention.
A. Suspensions for Nebulisation
a)

| | |
|---|---|
| Compound of Example 1, sterile | 1.0 mg |
| Water for Injections | to 10.0 ml |

Disperse the naphthoquinone in the Water for Injections previously sterilised in a sterile container. Fill in to sterile glass ampoules, 10 ml/ampoule under aseptic conditions, and seal each ampoule by fusion of the glass.
b) The following suspension was prepared:

| | |
|---|---|
| Compound of Example 1, micronised | 1.0 g |
| Polysorbate 20 | 0.1% w/v |
| Water for Injections | to 10 ml |

The Polysorbate 20 was dispersed in the water for injections, followed by the compound of Example 1. This suspension was filled into sterile glass ampoules, 10ml/ampoule under aspetic conditions, and the ampoules sealed by fusion of the glass.
B. Aerosol Formulations
a)

| | |
|---|---|
| Compound of Example 1, micronised | 1.0 mg |
| Aerosol propellant | to 5.0 ml |

Suspend the micronised naphthoquinone in the aerosol propellant. Fill this suspension into preformed aerosol cannisters, 5 ml/cannister under pressure, through the valve orifice.
b)

| | |
|---|---|
| Compound of Example 1, micronised | 1.0 mg |
| Arlacel 85 | 0.1% w/v |
| Aerosol propellant | to 5 ml |

Disperse the Arlacel 85 in the aerosol propellant and then add compound of Example 1. Fill the suspension into preformed aerosol cannisters, 5ml/cannister under pressure, through the valve orifice.
C. Powder Inhalation

| | |
|---|---|
| Compound of Example 1, micronised | 1.0 mg |
| Lactose | 29.0 mg |

Triturate and blend the micronised naphthoquinone with the lactose. Fill the resulting powder blend into hard gelatin capsule shells, 30 mg per capsule.
D. Nasal Drops

| | |
|---|---|
| Compound of Example 1 | 100.0 mg |
| Methylhydroxybenzoate | 10.0 mg |
| Water for Injections | to 10.0 ml |

Disperse the naphthoquinone and the methylhydroxybenzoate in the Water for Injections. Fill this suspension into suitable dropper bottles, 10 ml/bottle, and close by securing the dropper nozzle and bottle cap.

### Example 7

### Antimalarial Activity of Compounds (VI)

### Test methods

### Activity against Plasmodium Falciparum in vitro

The test method was modification of that described by Desjardins et al., Antimicrob. Agents and Chemotherapy, 1979, 16, 710-718. Compounds were dissolved in ethanol at a concentration of 4.8x10⁻³M and dilutions down to 1x10⁻⁴M were made. The drug solutions were serially diluted using RPMI 1640 medium + 10% human plasma in microtitration plates. Parasitised and fresh red blood cells were added, together with G-³H-hypoxanthine, in RPMI 1640 medium + 10% human plasma and the cultures incubated for 48 hours. Cultures were then harvested, the particulate contents collected on a glass fibre filter paper and washed copiously with water. The filter papers were dried and the radioactivity measured using a scintillation counter. Infected untreated and uninfected untreated cultures were included as controls.

The results are shown in Table 1.

### Activity against Plasmodium yoelii in vivo

The naphthoquinone was suspended in 0.25% (w/v) celacol in water by milling for 16-24 hours at 26°C. The suspensions were subsequently serially diluted with 0.25% (w/v) celacol in water.

At time 0, 0.1 ml of a suspension of 5x10⁶ P.yoelii-parasitised red blood cells/ml of phosphate saline were injected intravenously into 15-20 g mice through a tail vein. Groups of 5 mice per treatment were dosed orally at times 6, 22, 30, 46. 54. 70 and 78 hours with 0.2 ml of the drug suspension. Tail-blood smears were taken at 96 hours. stained with Giemsa and the percentage of red blood cells infected determined and compared to untreated, infected controls. Percent inhibition was correllated with dose to provide ED values. The results are shown in Table 1 below.

**TABLE 1 -**

| Antimalarial activity in vitro and in vivo | | | |
|---|---|---|---|
| Compound of Example No: | In vitro IC₅₀(µM) | In vivo ED₅₀ mg/kg. | |
| | | oral | i.v. |
| 1C | 0.002 | 0.03 | |
| | | | |
| 2 | 0.068 | 0.12 | |
| | | | |
| 3 | 0.080 | 0.09 | |
| | | | |
| 4 | 0.22 | 0.12 | |

## Claims (Claims for the following Contracting State(s): AT, BE, CH, LI, DE, FR, GB, IT, LU, NL, SE)

1. Novel compounds of formula (VI) 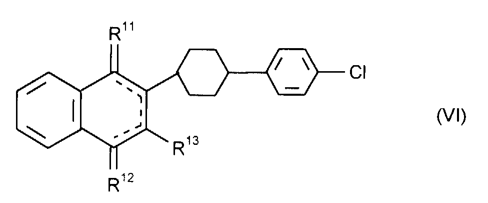 wherein
R¹¹ and R¹² each represent =O and the dotted line represents a double bond between the 2 and 3 positions of the quinone ring, in which case R¹³ represents a group OCOR⁵, wherein R⁵ is a C₁₋₁₀alkyl group, a C₃₋₁₀ cycloalkyl group, a C₁₋₁₀alkoxy group, or a phenyl or naphthyl group, each such R⁵ group being optionally substituted e.g. by amino, mono-or di-C₁₋₄alkylamino, carboxy or hydroxy;
a group OR⁶ or SR⁶, wherein R⁶ is an optionally substituted C₁₋₁₀alkyl, C₃₋₁₀ cycloalkyl, phenyl or naphthyl group as defined for R⁵; or
a group NR⁷R⁸, wherein R⁷ and R⁸ each independently represent hydrogen or C₁₋₄alkyl, or the group NR⁷R⁸ represents a 5-7 membered saturated heterocyclic ring, which may optionally contain a further heteroatom selected from nitrogen, oxygen or sulphur;
or the dotted line represents double bonds at the 1,2 and 3,4 positions of the quinol ring and R¹¹, R¹² and R¹³ each represents a group -OCOR¹⁴, wherein R¹⁴ represents an optionally substituted C₁₋₁₀alkyl group.

2. A pharmaceutical formulation which comprises a compound of formula (VI) in association with a pharmaceutically acceptable carrier therefor.

3. A process for preparing a compound of formula (VI) as defined in claim 1 which comprises:
(a) reaction of a compound of formula (V) or (VII) 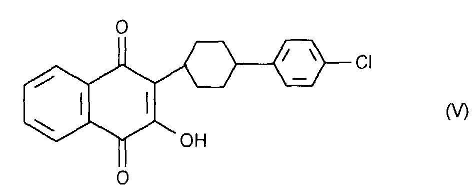 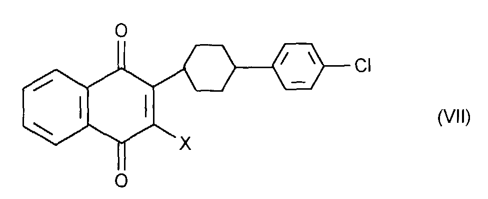 wherein X is a halogen atom, with a compound serving to introduce the required group R¹³, and if necessary and/or desired the groups R¹¹ and R¹²; or
(b) reaction of a compound of formula (VIII) 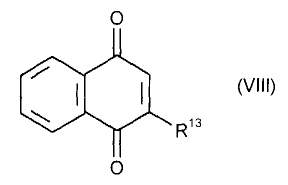 with a donor compound serving to introduce the 4-(4-chlorophenyl)cyclohexyl group.

4. A compound of formula (VI) as claimed in claim 1 for use in therapy.

5. Use of a compound of formula (VI) as claimed in claim 1 for the manufacture of a medicament for the treatment and/or prophylaxis of P.Carinii infections.

6. Use of a compound of formula (VI) as claimed in claim 1 for the manufacture of a medicament for the treatment and/or prophylaxis of malaria.

## Claims (Claims for the following Contracting State(s): ES, GR)

1. Novel compounds of formula (VI) 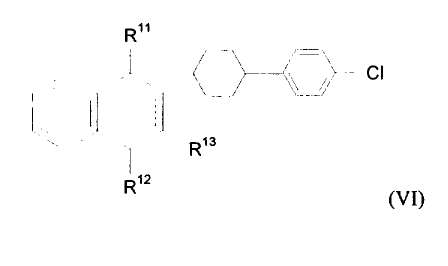 wherein
R¹¹ and R¹² each represent =O and the dotted line represents a double bond between the 2 and 3 positions of the quinone ring, in which case R¹³ represents a group OCOR⁵, wherein R⁵ is a C₁₋₁₀alkyl group, a C₃₋₁₀ cycloalkyl group, a C₁₋₁₀alkoxy group, or a phenyl or naphthyl group, each such R⁵ group being optionally substituted e.g. by amino, mono-or di-C₁₋₄alkylamino, carboxy or hydroxy;
a group OR⁶ or SR⁶, wherein R⁶ is an optionally substituted C₁₋₁₀alkyl, C₃₋₁₀ cycloalkyl, phenyl or naphthyl group as defined for R⁵; or
a group NR⁷R⁸, wherein R⁷ and R⁸ each independently represent hydrogen or C₁₋₄alkyl, or the group NR⁷R⁸ represents a 5-7 membered saturated heterocyclic ring, which may optionally contain a further heteroatom selected from nitrogen, oxygen or sulphur;
or the dotted line represents double bonds at the 1,2 and 3,4 positions of the quinol ring and R¹¹, R¹² and R¹³ each represents a group -OCOR¹⁴, wherein R¹⁴ represents an optionally substituted C₁₋₁₀alkyl group.

2. A pharmaceutical formulation which comprises a compound of formula (VI) in association with a pharmaceutically acceptable carrier therefor.

3. A process for preparing a compound of formula (VI) as defined in claim 1 which comprises:
(a) reaction of a compound of formula (V) or (VII) 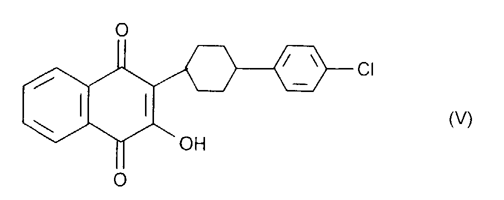 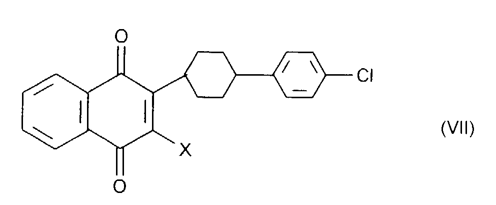 wherein X is a halogen atom, with a compound serving to introduce the required group R¹³, and if necessary and/or desired the groups R¹¹ and R¹²; or
(b) reaction of a compound of formula (VIII) 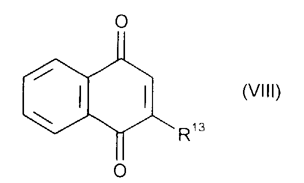 with a donor compound serving to introduce the 4-(4-chlorophenyl)cyclohexyl group.

4. A compound of formula (VI) as claimed in claim 1 for use in therapy.

5. Use of a compound of formula (VI) as claimed in claim 1 for the manufacture of a medicament for the treatment and/or prophylaxis of P.Carinii infections.

6. Use of a compound of formula (VI) as claimed in claim 1 for the manufacture of a medicament for the treatment and/or prophylaxis of malaria.

## Patentansprüche (Patentansprüche für folgende(n) Vertragsstaat(en): AT, BE, CH, LI, DE, FR, GB, IT, LU, NL, SE)

1. Neue Verbindungen der Formel (VI): 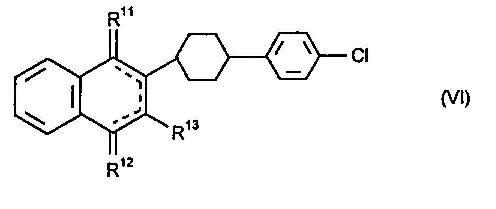 worin,
R¹¹ und R¹² jeweils =O darstellen und die gestrichelte Linie eine Doppelbindung zwischen den 2- und 3-Positionen des Chinon-Rings darstellt, wobei in diesem Fall R¹³ darstellt:
eine Gruppe OCOR⁵, worin R⁵ eine C₁₋₁₀-Alkyl-Gruppe, eine C₃₋₁₀-Cycloalkyl-Gruppe, eine C₁₋₁₀-Alkoxy-Gruppe oder eine Phenyl- oder Naphthyl-Gruppe ist, wobei jede solche R⁵-Gruppe gegebenenfalls substituiert ist, z.B. mit Amino, Mono- oder Di-C₁₋₄-alkylamino, Carboxy oder Hydroxy; oder
eine Gruppe OR⁶ oder SR⁶, worin R⁶ eine gegebenenfalls substituierte C₁₋₁₀-Alkyl-, C₃₋₁₀-Cycloalkyl-, Phenyl- oder Naphthyl-Gruppe ist, wie für R⁵ definiert; oder eine Gruppe NR⁷R⁸, worin R⁷ und R⁸ jeweils unabhängig Wasserstoff oder C₁₋₄-Alkyl darstellen, oder worin die Gruppe NR⁷R⁸ einen 5-7-gliedrigen gesättigten heterocyclischen Ring darstellt, der gegebenenfalls ein zusätzliches Heteroatom enthalten kann, ausgewählt aus Stickstoff, Sauerstoff oder Schwefel;
oder die gestrichelte Linie Doppelbindungen in den 1,2- und 3,4-Positionen des Hydrochinon-Rings darstellt und R¹¹, R¹² und R¹³ jeweils eine Gruppe -OCOR¹⁴ darstellen, worin R¹⁴ eine gegebenenfalls substituierte C₁₋₁₀-Alkyl-Gruppe darstellt.

2. Pharmazeutische Formulierung, die eine Verbindung der Formel (VI) in Verbindung mit einem pharmazeutisch akzeptablen Träger dafür umfaßt.

3. Verfahren zur Herstellung einer Verbindung der Formel (VI) wie in Anspruch 1 definiert, welches umfaßt:
(a) Reaktion einer Verbindung der Formel (V) oder (VII): 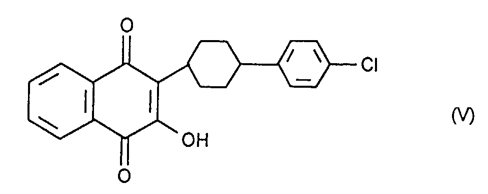 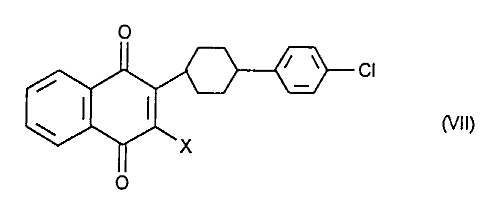 worin X ein Halogenatom ist, mit einer Verbindung, die dazu dient, die erforderliche Gruppe R¹³ und, falls notwendig und/oder gewünscht, die Gruppen R¹¹ und R¹² einzuführen; oder
(b) Reaktion einer Verbindung der Formel (VIII): 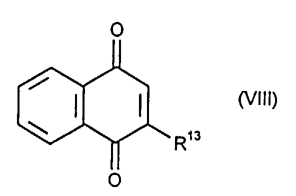 mit einer Donor-Verbindung, die dazu dient, die 4-(4-Chlorphenyl)cyclohexyl-Gruppe einzuführen.

4. Verbindung der Formel (VI) wie in Anspruch 1 beansprucht zur Verwendung in der Therapie.

5. Verwendung einer Verbindung der Formel (VI) wie in Anspruch 1 beansprucht zur Herstellung eines Arzneimittels zur Behandlung und/oder Prophylaxe von P. carinii-Infektionen.

6. Verwendung einer Verbindung der Formel (VI) wie in Anspruch 1 beansprucht zur Herstellung eines Arzneimittels zur Behandlung und/oder Prophylaxe von Malaria.

## Patentansprüche (Patentansprüche für folgende(n) Vertragsstaat(en): ES, GR)

1. Neue Verbindungen der Formel (VI): 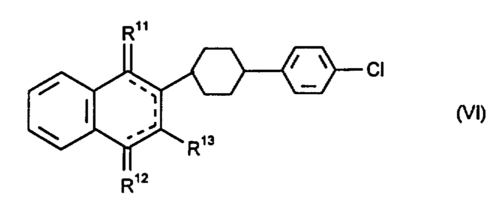 worin,
R¹¹ und R¹² jeweils =O darstellen und die gestrichelte Linie eine Doppelbindung zwischen den 2- und 3-Positionen des Chinon-Rings darstellt, wobei in diesem Fall R¹³ darstellt:
eine Gruppe OCOR⁵, worin R⁵ eine C₁₋₁₀-Alkyl-Gruppe, eine C₃₋₁₀-Cycloalkyl-Gruppe, eine C₁₋₁₀-Alkoxy-Gruppe oder eine Phenyl- oder Naphthyl-Gruppe ist, wobei jede solche R⁵-Gruppe gegebenenfalls substituiert ist, z.B. mit Amino, Mono- oder Di-C₁₋₄-alkylamino, Carboxy oder Hydroxy; oder
eine Gruppe OR⁶ oder SR⁶, worin R⁶ eine gegebenenfalls substituierte C₁₋₁₀-Alkyl-, C₃₋₁₀-Cycloalkyl-, Phenyl- oder Naphthyl-Gruppe ist, wie für R⁵ definiert; oder eine Gruppe NR⁷R⁸, worin R⁷ und R⁸ jeweils unabhängig Wasserstoff oder C₁₋₄-Alkyl darstellen, oder worin die Gruppe NR⁷R⁸ einen 5-7-gliedrigen gesättigten heterocyclischen Ring darstellt, der gegebenenfalls ein zusätzliches Heteroatom enthalten kann, ausgewählt aus Stickstoff, Sauerstoff oder Schwefel;
oder die gestrichelte Linie Doppelbindungen in den 1,2- und 3,4-Positionen des Hydrochinon-Rings darstellt und R¹¹, R¹² und R¹³ jeweils eine Gruppe -OCOR¹⁴ darstellen, worin R¹⁴ eine gegebenenfalls substituierte C₁₋₁₀-Alkyl-Gruppe darstellt.

2. Pharmazeutische Formulierung, die eine Verbindung der Formel (VI) in Verbindung mit einem pharmazeutisch akzeptablen Träger dafür umfaßt.

3. Verfahren zur Herstellung einer Verbindung der Formel (VI) wie in Anspruch 1 definiert, welches umfaßt:
(a) Reaktion einer Verbindung der Formel (V) oder (VII): 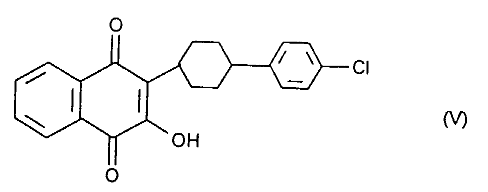 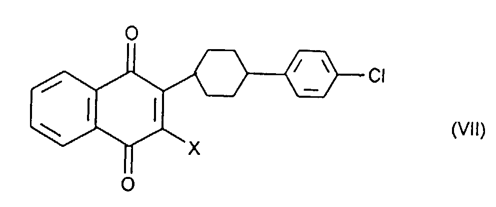 worin X ein Halogenatom ist, mit einer Verbindung, die dazu dient, die erforderliche Gruppe R¹³ und, falls notwendig und/oder gewünscht, die Gruppen R¹¹ und R¹² einzuführen; oder
(b) Reaktion einer Verbindung der Formel (VIII): 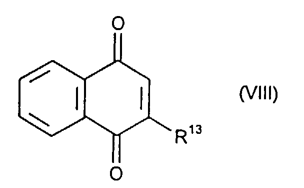 mit einer Donor-Verbindung, die dazu dient, die 4-(4-Chlorphenyl)cyclohexyl-Gruppe einzuführen.

4. Verbindung der Formel (VI) wie in Anspruch 1 beansprucht zur Verwendung in der Therapie.

5. Verwendung einer Verbindung der Formel (VI) wie in Anspruch 1 beansprucht zur Herstellung eines Arzneimittels zur Behandlung und/oder Prophylaxe von P. carinii-Infektionen.

6. Verwendung einer Verbindung der Formel (VI) wie in Anspruch 1 beansprucht zur Herstellung eines Arzneimittels zur Behandlung und/oder Prophylaxe von Malaria.

## Revendications (Revendications pour l'(les) Etat(s) contractant(s) suivant(s): AT, BE, CH, LI, DE, FR, GB, IT, LU, NL, SE)

1. Nouveaux composés de formule (VI) : 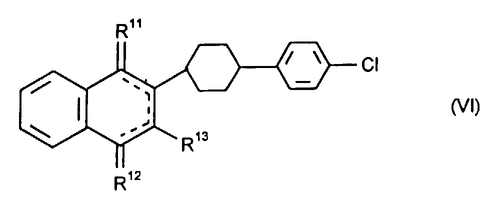 dans laquelle :
R¹¹ et R¹² représentent chacun =O et le trait en pointillé représente une double liaison entre les positions 2 et 3 du noyau quinone, auquel cas R¹³ représente un groupe OCOR⁵, dans lequel R⁵ est un groupe alkyle en C₁₋₁₀, un groupe cycloalkyle en C₃₋₁₀, un groupe alcoxy en C₁₋₁₀, ou un groupe phényle ou naphtyle, chacun de ces groupes R⁵ étant éventuellement substitué par exemple par un groupe amino, mono- ou dialkyl(C₁₋₄)amino, carboxy ou hydroxy;
un groupe OR⁶ ou SR⁶, dans lequel R⁶ est un groupe alkyle en C₁₋₁₀, cycloalkyle en C₃₋₁₀, phényle ou naphtyle éventuellement substitué tel que défini pour R⁵; ou
un groupe NR⁷R⁸, dans lequel R⁷ et R⁸ représentent chacun indépendamment de l'hydrogène ou un groupe alkyle en C₁₋₄, ou bien le groupe NR⁷R⁸ représente un noyau hétérocyclique saturé de 5 à 7 chaînons, qui peut éventuellement contenir un hétéroatome supplémentaire choisi parmi l'azote, l'oxygène et le soufre;
ou bien la ligne en pointillé représente des doubles liaisons aux positions 1,2 et 3,4 du noyau quinol et R¹¹, R¹² et R¹³ représentent chacun un groupe -OCOR¹⁴, dans lequel R¹⁴ représente un groupe alkyle en C₁₋₁₀ éventuellement substitué.

2. Formulation pharmaceutique qui comprend un composé de formule (VI) en association avec un support pharmaceutiquement acceptable.

3. Procédé de préparation d'un composé de formule (VI) suivant la revendication 1, qui comprend :
(a) la réaction d'un composé de formule (V) ou (VII) : 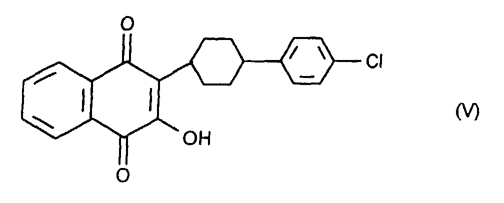 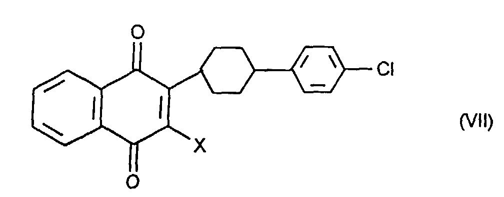 dans laquelle X est un atome d'halogène, avec un composé servant à introduire le groupe R¹³ requis, et si nécessaire et/ou désirable les groupes R¹¹ et R¹²; ou
(b) la réaction d'un composé de formule (VIII) : 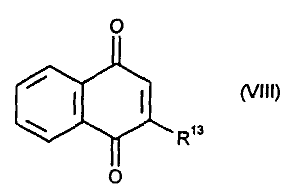 avec un composé donneur servant à introduire le groupe 4-(4-chlorophényl)cyclohexyle.

4. Composé de formule (VI) suivant la revendication 1 destiné à être utilisé en thérapie.

5. Utilisation d'un composé de formule (VI) suivant la revendication 1 pour la fabrication d'un médicament pour le traitement et/ou la prophylaxie d'infections dues à P. carinii.

6. Utilisation d'un composé de formule (VI) suivant la revendication 1 pour la fabrication d'un médicament pour le traitement et/ou la prophylaxie de la malaria.

## Revendications (Revendications pour l'(les) Etat(s) contractant(s) suivant(s): ES, GR)

1. Nouveaux composés de formule (VI) : 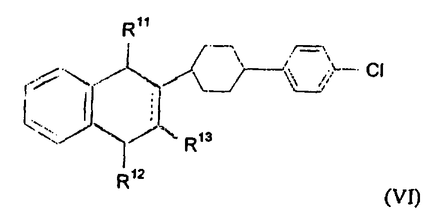 dans laquelle :
R¹¹ et R¹² représentent chacun =O et le trait en pointillé représente une double liaison entre les positions 2 et 3 du noyau quinone, auquel cas R¹³ représente un groupe OCOR⁵, dans lequel R⁵ est un groupe alkyle en C₁₋₁₀, un groupe cycloalkyle en C₃₋₁₀, un groupe alcoxy en C₁₋₁₀, ou un groupe phényle ou naphtyle, chacun de ces groupes R⁵ étant éventuellement substitué par exemple par un groupe amino, mono- ou dialkyl(C₁₋₄)amino, carboxy ou hydroxy;
un groupe OR⁶ ou SR⁶, dans lequel R⁶ est un groupe alkyle en C₁₋₁₀, cycloalkyle en C₃₋₁₀,phényle ou naphtyle éventuellement substitué tel que défini pour R⁵; ou
un groupe NR⁷R⁸, dans lequel R⁷ et R⁸ représentent chacun indépendamment de l'hydrogène ou un groupe alkyle en C₁₋₄, ou bien le groupe NR⁷R⁸ représente un noyau hétérocyclique saturé de 5 à 7 chaînons, qui peut éventuellement contenir un hétéroatome supplémentaire choisi parmi l'azote, l'oxygène et le soufre;
ou bien la ligne en pointillé représente des doubles liaisons aux positions 1,2 et 3,4 du noyau quinol et R¹¹, R¹² et R¹³ représentent chacun un groupe -OCOR¹⁴, dans lequel R¹⁴ représente un groupe alkyle en C₁₋₁₀ éventuellement substitué.

2. Formulation pharmaceutique qui comprend un composé de formule (VI) en association avec un support pharmaceutiquement acceptable.

3. Procédé de préparation d'un composé de formule (VI) suivant la revendication 1, qui comprend :
(a) la réaction d'un composé de formule (V) ou (VII) : 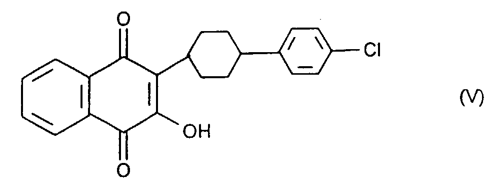 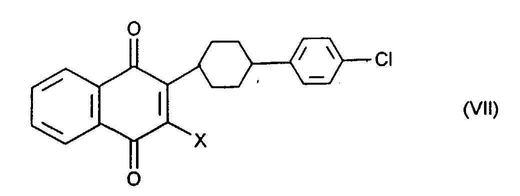 dans laquelle X est un atome d'halogène, avec un composé servant à introduire le groupe R¹³ requis, et si nécessaire et/ou désirable les groupes R¹¹ et R¹²; ou
(b) la réaction d'un composé de formule (VIII) : 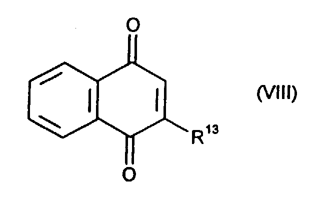 avec un composé donneur servant à introduire le groupe 4-(4-chlorophényl)cyclohexyle.

4. Composé de formule (VI) suivant la revendication 1 destiné à être utilisé en thérapie.

5. Utilisation d'un composé de formule (VI) suivant la revendication 1 pour la fabrication d'un médicament pour le traitement et/ou la prophylaxie d'infections dues à P. carinii.

6. Utilisation d'un composé de formule (VI) suivant la revendication 1 pour la fabrication d'un médicament pour le traitement et/ou la prophylaxie de la malaria.
